(19)

Europäisches
Patentamt
European
Patent Office
Office européen
des brevets

(11) **EP 4 449 988 A1**

(12) **EUROPEAN PATENT APPLICATION**
published in accordance with Art. 153(4) EPC

(43) Date of publication:
**23.10.2024 Bulletin 2024/43**

(21) Application number: **22907246.7**

(22) Date of filing: **02.12.2022**

(51) International Patent Classification (IPC):
**A61B 5/107** *(2006.01)*      **A61B 5/00** *(2006.01)*
**A61B 8/14** *(2006.01)*

(52) Cooperative Patent Classification (CPC):
**A61B 5/00; A61B 5/107; A61B 8/14**

(86) International application number:
**PCT/JP2022/044536**

(87) International publication number:
**WO 2023/112718 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.12.2021 JP 2021205185**

(71) Applicant: **Shiseido Company, Ltd.**
**Chuo-ku**
**Tokyo 104-0061 (JP)**

(72) Inventor: **EZURE, Tomonobu**
**Tokyo 104-0061 (JP)**

(74) Representative: **Cabinet Beau de Loménie**
**158, rue de l'Université**
**75340 Paris Cedex 07 (FR)**

(54) **WRINKLE EVALUATION METHOD, WRINKLE EVALUATION SYSTEM, FAT INFILTRATION EVALUATION METHOD, FAT INFILTRATION EVALUATION SYSTEM, COMPUTER PROGRAM, SKIN SENSING METHOD FOR WRINKLE EVALUATION PURPOSES, RECOMMENDATION METHOD ON BASIS OF WRINKLE EVALUATION, AND METHOD FOR PREDICTING FUTURE STATE OF FAT INFILTRATION AND/OR FUTURE STATE OF WRINKLES**

(57)    The present disclosure provides a method for evaluating wrinkles on the forehead of a subject. The method for evaluating wrinkles uses the state of cavitation (fat infiltration) in the dermis of the forehead of the subject as the basis for assessment. It is evaluated that the risk of wrinkle formation is high or that wrinkles are numerous when cavitation is found to have occurred in the dermis of the forehead of the subject. The wrinkles may be expression (transient) wrinkles and/or fixed wrinkles. Also provided is a method for predicting the future state of wrinkles based on the state of cavitation.

Fig. 2

$$Y = -0.1 X + 1.5$$
$$R = -0.6 \ (P<0.001)$$

EP 4 449 988 A1

**Description**

TECHNICAL FIELD

The present invention relates to a method for evaluating wrinkles in human skin, and more specifically it relates to a method for evaluating wrinkles whereby the state of cavitation (fat infiltration) in the dermis of the forehead is used as the basis for assessment.

BACKGROUND

Skin aging is the alteration of skin caused by numerous factors including internal factors such as aging, hormones and metabolism, and external factors such as ultraviolet rays and drying. Skin aging can usually be observed based on exterior appearances such as skin spots, wrinkles, sagging and shrinkage, but aging also proceeds with non-visible changes in the interior skin tissue structure and its constituent components. In PTL 1, for example, aging is assessed based on blood vitamin D levels. In PTL 2, expression of genes such as the E-cadherin gene and T-cadherin gene in skin is used as an index. In PTL 3, expression of genes such as OLFML2A and/or CRLF1 in fibroblasts is used as the index.

Although several indicators exist for assessing skin aging, it is important to achieve even more proper evaluation of skin condition and to adopt countermeasures to prevent or improve its aging, and therefore more methods are still desired for evaluation of skin condition.

PTL 4 discloses dermal cavitation as one cause of sagging of cheek skin, and further discloses an ameliorator for skin sagging or aging caused by dermal cavitation, which includes an adipose stem cell inducer comprising licorice extract or rosemary extract.

CITATION LIST

PATENT LITERATURE

PTL 1 Japanese Unexamined Patent Publication No. 2016-216435
PTL 2 Japanese Unexamined Patent Publication No. 2010-115131
PTL 3 Japanese Unexamined Patent Publication No. 2013-116088
PTL 4 WO2017/022091

SUMMARY

TECHNICAL PROBLEM

One object of the disclosure is to provide a method for evaluating wrinkles wherein the state of cavitation (fat infiltration) in the dermis of the forehead of a subject is used as the basis for assessment, and in particular to provide a method for evaluating transient expression of wrinkles which are not easy to evaluate based on outer appearance. Transiently appearing expression wrinkles are a major factor in the appearance of human aging, and are the primary cause of formation of established wrinkles. As used herein, the term "transient (expression) wrinkles" will be used to refer to wrinkles transiently formed by changes in facial expression. Wrinkles resulting with establishment of transient wrinkles, even without changes in facial expression, will be referred to as "fixed transient wrinkles" or "fixed wrinkles". Wrinkles due to facial expressions are formed from both transient wrinkles and fixed transient wrinkles. Another object of the disclosure is to provide a system for evaluating wrinkles, which evaluates wrinkles including not only fixed wrinkles that can be easily measured based on outer appearance, but also transient wrinkles, with the state of cavitation (fat infiltration) in the dermis of the forehead of a subject as the basis for assessment.

SOLUTION TO PROBLEM

The present inventors have found that it is possible to evaluate wrinkles, and especially transient wrinkles which are not easy to evaluate based on outer appearance, using the state of cavitation (fat infiltration) in the dermis of the forehead of a subject as the basis for assessment. The present disclosure therefore provides a method and system for evaluating wrinkles, comprising not only fixed wrinkles but also transient wrinkles, using the state of cavitation (fat infiltration) in the dermis of the forehead as the basis for assessment. More specifically, the present disclosure relates to the following aspects:

[Aspect 1]

A method for evaluating wrinkles, wherein the state of cavitation (fat infiltration) in the dermis of the forehead of a subject is used as the basis for assessment.

[Aspect 2]

The method according to aspect 1, comprising a step of measuring the state of cavitation by echo or laser microscope.

[Aspect 3]

The method according to aspect 1 or 2, wherein it is evaluated that the risk of wrinkle formation is high or that wrinkles are numerous when cavitation has occurred.

[Aspect 4]

The method according to any one of aspects 1 to 3, wherein the wrinkles are transient wrinkles and/or fixed wrinkles.

[Aspect 5]

The method according to any one of aspects 1 to 4, wherein cavitation is evaluated by the distance from the skin surface to a cavity, as measured by echo or laser microscope.

[Aspect 6]

A system for evaluating wrinkles, wherein the wrinkles are evaluated using the state of cavitation (fat infiltration) in the dermis of the forehead of a subject as the basis for assessment.

[Aspect 7]

The system according to aspect 6, which comprising:

a measuring unit that measures the state of cavitation in the dermis of the forehead of the subject,
an analysis unit that analyzes the measured state of cavitation,
a wrinkle evaluating unit that evaluates wrinkles based on the analysis results and
a results output unit that outputs the evaluation results.

[Aspect 8]

The system according to aspect 7, wherein the measuring unit is configured so as to measure the state of cavitation by echo or laser microscope.

[Aspect 9]

The system according to aspect 7 or 8, wherein the wrinkle evaluating unit carries out evaluation by comparing the measured value with a predetermined reference value.

[Aspect 10]

A computer program for executing the method according to any one of aspects 1 to 5 in a system according to any one of aspects 7 to 9.

[Aspect 11]

A method for evaluating cavitation (fat infiltration), wherein the state of wrinkles on the forehead of a subject is used as the basis for assessment.

[Aspect 12]

The method according to aspect 11, wherein the wrinkles are transient wrinkles and/or fixed wrinkles.

[Aspect 13]

The method according to aspect 11 or 12, wherein it is evaluated that cavitation has occurred when wrinkles have formed.

[Aspect 14]

A system for evaluating cavitation (fat infiltration), wherein the state of cavitation is evaluated using the state of wrinkles on the forehead of a subject as the basis for assessment.

[Aspect 15]

The system according to aspect 14, comprising:

an image acquisition unit that acquires an image of wrinkles on the forehead of a subject,
an analysis unit that analyzes the acquired image,
a cavitation evaluating unit that evaluates the state of cavitation based on the analysis results and
a results output unit that outputs the evaluation results.

[Aspect 16]

The system according to aspect 15, wherein the cavitation evaluating unit carries out evaluation by comparing the measured value with a predetermined reference value.

[Aspect 17]

A computer program for executing the method according to any one of aspects 11 to 13 in a system according to any one of aspects 14 to 16.

[Aspect 18]

A skin sensing method for evaluation of wrinkles, wherein the state of cavitation (fat infiltration) in the dermis of the forehead of a subject is included as a measured parameter.

[Aspect 19]

A recommendation method based on evaluation of wrinkles using sensing data obtained by the method according to aspect 18.

[Aspect 20]

A method for predicting future cavitation state and/or wrinkle state, based on the state of cavitation (fat infiltration) in the dermis of the forehead of a subject at a measuring time point.

BRIEF DESCRIPTION OF DRAWINGS

Fig. 1 is an echo image showing dermis without cavitation (left) and dermis with cavitation (right).
Fig. 2 is a graph showing the correlation between transient wrinkles and degree of cavitation.
Fig. 3 is a graph showing the correlation between transient wrinkles and degree of fixed wrinkles.
Fig. 4 is a graph showing the correlation between fixed wrinkles and degree of cavitation.
Fig. 5 shows an example of the construction of the system of the disclosure.
Fig. 6 shows an example of the construction of the system of the disclosure.

Fig. 7 shows an example of the construction of the system of the disclosure.
Fig. 8 is a diagram showing an example of a hardware configuration capable of carrying out processing according to the disclosure.
Fig. 9 is a flow chart showing an example of processing (a program) carried out by the system of the disclosure.
Fig. 10 is a flow chart showing an example of processing (a program) carried out by the system of the disclosure.

DESCRIPTION OF EMBODIMENTS

Method for evaluating wrinkles

One aspect of the disclosure provides a method for evaluating wrinkles wherein the state of cavitation (fat infiltration) in the dermis of the forehead of a subject is used as the basis for assessment, and more particularly it provides a method for evaluating expression wrinkles which are not easy to evaluate based on outer appearance. Transient wrinkles are wrinkles transiently formed due to facial expression muscles. For example, forehead wrinkles can form due to sagging or drooping of the eyelids. With impaired field of view due to sagging or drooping of eyelids, the forehead muscles attempt to open the eyes, causing transient wrinkles on the forehead. When transient wrinkles are left untreated, the transient wrinkles eventually become fixed wrinkles, leaving wrinkles even in the absence of the facial expression. As mentioned above, the present inventors have found that wrinkles can be evaluated by examining the state of cavitation in the dermis of the forehead of a subject. For example, the state of cavitation can be measured by echo (ultrasound) or laser microscope methods. Transiently appearing wrinkles have previously been difficult to measure and evaluate, but the method of the disclosure allows not only fixed wrinkles but also transient wrinkles to be conveniently evaluated by measuring dermal cavitation.

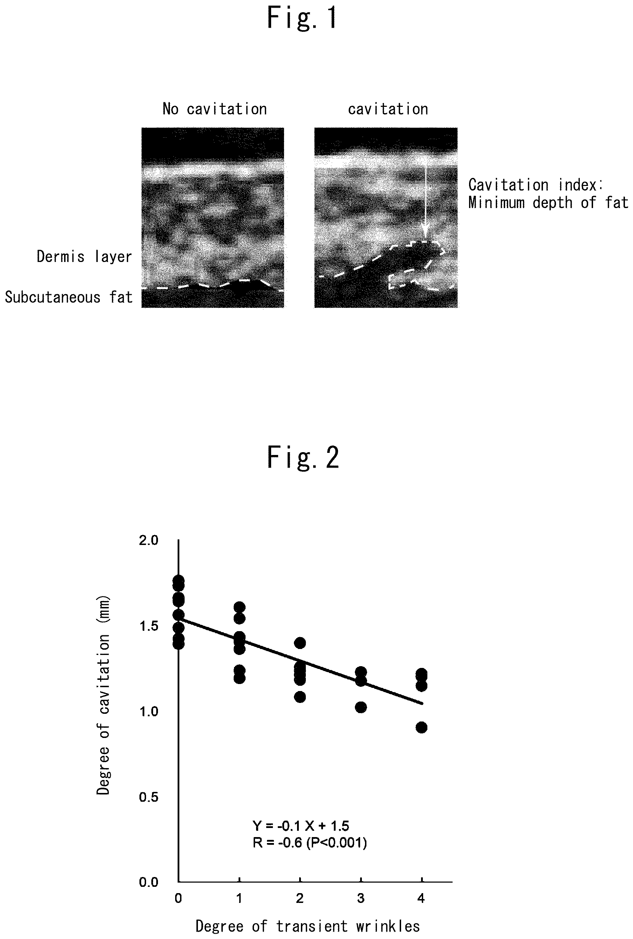

Dermal cavities are cavities formed by partial rising of subcutaneous tissue on the dermis layer side, by aging or by some other cause (Fig. 1). The sizes of dermal cavities range from 10 $\mu$m to 1000 $\mu$m in diameter with approximately spherical shapes, though the shapes are not consistent. Because the dermal cavity interiors are filled by subcutaneous tissue, they may be referred to as "filled dermal cavities". Since cellular components and interstitial components in the dermis layer are not found in dermal cavities, they form wrinkles. According to one aspect of the disclosure, it is evaluated that the risk of wrinkle formation is high or that wrinkles are numerous when cavitation has occurred. For example, when cavitation has occurred, even if no wrinkles are observed, the potential for transient wrinkle formation is normally high with changes in facial expression, and such transient wrinkles are highly likely to progress to fixed wrinkles in the future.

The extent of dermal cavities can be measured by estimating the dermal thickness, i.e. the thickness from the boundary with the epidermis to the boundary with the subcutaneous tissue, in a fixed-length skin cross-section. When dermal cavities are present, the subcutaneous tissue rises to the dermis layer side, and the thickness of the dermis decreases. Since the thickness of the epidermis is normally constant and somewhat thinner compared to the dermis, it can be calculated by simply estimating the thickness from the skin surface to the subcutaneous tissue. According to several aspects of the disclosure, therefore, cavitation can be evaluated by the distance from the skin surface to a cavity, as measured by echo or laser microscope. Because the extent of dermal cavities is correlated with elasticity of skin, an index of skin elasticity such as the Uv/Ue value may be measured instead, using a device that measures skin elasticity, such as a Cutometer.

According to several aspects of the disclosure, wrinkles may be transient wrinkles and/or fixed wrinkles. Transient wrinkles are wrinkles transiently formed due to facial expression muscles. For example, forehead wrinkles can form due to sagging or drooping of the eyelids. With impaired field of view due to sagging or drooping of eyelids, the forehead muscles attempt to open the eyes, causing transient wrinkles on the forehead. When transient wrinkles are left untreated, the transient wrinkles eventually become fixed wrinkles (also known as established wrinkles), leaving wrinkles even in the absence of the facial expression. Since transient wrinkles are transient with changes in facial expression and do not permanently remain, their measurement and evaluation has been difficult in the past.

According to one aspect of the disclosure, it is evaluated that the risk of transient wrinkle formation is high or that transient wrinkles are numerous when cavitation has occurred. The method of the disclosure allows the extent of wrinkles to be calculated based on the extent of cavitation. A high risk of transient wrinkles or numerous transient wrinkles may be associated with future evaluation a high risk of fixed wrinkles or numerous fixed wrinkles. According to one aspect of the disclosure, therefore, it is evaluated that the risk of fixed wrinkle formation is high or that fixed wrinkles are numerous when cavitation has occurred. In other words, the method of the disclosure is useful not only for evaluation of the current state of wrinkles but also for prediction of the future state of wrinkles.

[Future prediction]

One aspect of the disclosure relates to a method for predicting future cavitation state and/or wrinkle state, based on the state of cavitation (fat infiltration) in the dermis of the forehead of a subject at a measuring time point. The present inventors have found the following correlation between cavitation and age.

$$Y = -0.02X + 2.2 \ (P < 0.01) \quad \text{Formula 1}$$

Here, X represents age or elapsed years, Y represents degree of cavitation (distance (mm) to the epidermis, with a smaller number indicating greater cavitation). Using the coefficient -0.02, the degree of cavitation after 10 years, for example, can be calculated as: (current degree of cavitation) - 0.02 ×10 years. Since the degree of wrinkles can also be calculated from the degree of cavitation, calculating the current state of cavitation allows the degree of transient wrinkles and fixed wrinkles after 10 years to be predicted.

One aspect of the disclosure, therefore, relates to a method for predicting the state of cavitation Y2 after X years, based on the state of cavitation (fat infiltration) in the dermis of the forehead of a subject at a measuring time point Y1, wherein Y2 is calculated based on the following Formula 2.

$$Y2 = Y1 - 0.02X \quad \text{Formula 2}$$

Based on the calculated values of Y2, or Y1 and X, the following formula derived from Fig. 2 may be used to also predict the degree of transient wrinkles Z1.

$$Z1 = -10Y2 + 15 = -10Y1 + 0.2X + 15 \quad \text{Formula 3}$$

One aspect of the disclosure, therefore, relates to a method for predicting the degree of transient wrinkles Z1 after X years, based on the state of cavitation (fat infiltration) in the dermis of the forehead of a subject at a measuring time point Y1, wherein Z1 is calculated based on Formula 3 above.

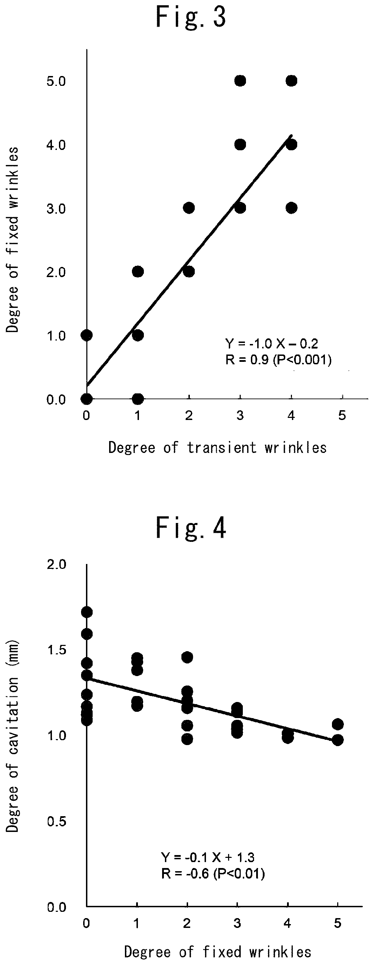

Likewise, based on the calculated values of Y2, or Y1 and X, the following formula derived from Fig. 4 may be used to also predict the degree of fixed wrinkles Z2.

$$Z2 = -10Y2 + 13 = -10Y1 + 0.2X + 13 \quad \text{Formula 4}$$

One aspect of the disclosure, therefore, relates to a method for predicting the degree of fixed wrinkles Z2 after X years, based on the state of cavitation (fat infiltration) in the dermis of the forehead of a subject at a measuring time point Y1, wherein Z2 is calculated based on Formula 4 above.

[Treatment of cavitation/wrinkles]

According to one aspect of the disclosure, when wrinkles have formed due to cavitation, a composition containing rosemary extract and/or licorice extract may be applied to the skin for treatment.

[Method for evaluating cavitation (fat infiltration)]

One aspect of the disclosure relates to a method for evaluating cavitation (fat infiltration), wherein the state of wrinkles on the forehead of a subject is used as the basis for assessment. As mentioned above, the present inventors have found that wrinkles can be evaluated by examining the state of cavitation in the dermis of the forehead of a subject. Conversely, this also means that cavitation can be evaluated using the state of wrinkles on the forehead of a subject as the basis for assessment. For example, the state of wrinkles can be evaluated using a Visia Evolution image analyzer by Canfield Scientific, or by replica analysis, photograph-based grade assessment, or visual assessment. For example, the state of wrinkles may be graded on a multi-level scale (such as 5 levels from 0 to 4).

According to a partial aspect of the disclosure, wrinkles are transient wrinkles and/or fixed wrinkles. According to a partial aspect of the disclosure, it may be evaluated that cavitation has occurred when transient wrinkles and/or fixed wrinkles have been formed.

According to one aspect of the disclosure, when cavitation has occurred in the forehead dermis, a composition containing rosemary extract and/or licorice extract may be applied to the skin for treatment.

[Sensing method]

One aspect of the disclosure relates to a skin sensing method for evaluation of wrinkles, wherein the state of cavitation (fat infiltration) in the dermis of the forehead of a subject is included as a measured parameter. Other measured parameters include, but are not limited to, parameters relating to skin color tone, moisture and elasticity. According to several aspects of the disclosure, the measured state of cavitation (fat infiltration) is visualized and displayed on a display device such as a liquid crystal display for presentation to the subject. The visualization may be carried out based on the results of future prediction of the state of cavitation or wrinkles. For example, cavitation in the dermis of the forehead of a customer may be measured (sensed) at a cosmetic retailer and the measurement results or prediction results displayed in a visualized manner as material for promoting a cosmetic product or cosmetic method to the customer. Several aspects of the disclosure therefore relate to a recommendation method based on evaluation of wrinkles using sensing data obtained by the method of the disclosure. Such recommendation may relate to a cosmetic product and/or a cosmetic method.

[Recommendation method]

One aspect of the disclosure relates to a method in which a subject is recommended an appropriate cosmetic, drug or cosmetic method based on the state of cavitation (fat infiltration) and wrinkles of the subject as evaluated by the method of the disclosure. For example, when an evaluation of high risk of wrinkles or numerous wrinkles has been obtained for the forehead of a subject by the method of the disclosure, the subject may be recommended the use of a cosmetic, drug or cosmetic method suited for improving wrinkles. When an evaluation of cavitation has been obtained for the forehead of a subject by the method of the disclosure, the subject may be recommended the use of a cosmetic, drug or cosmetic method suited for improving cavitation. The cosmetic, drug or cosmetic method recommended based on the condition of skin of the subject may also be recorded in a previously created database. A recommended cosmetic may be, for example, a product containing rosemary extract and/or licorice extract.

[Device and system for evaluation of wrinkles]

One aspect of the disclosure relates to a system for evaluating wrinkles, wherein the wrinkles are evaluated using the state of cavitation (fat infiltration) in the dermis of the forehead of a subject as the basis for assessment. According to a partial aspect, the system includes (i) a measuring unit that measures the state of cavitation in the dermis of the forehead of the subject, (ii) an analysis unit that analyzes the measured state of cavitation, (iii) a wrinkle evaluating unit that evaluates wrinkles based on the analysis results and (iv) a results output unit that outputs the evaluation results. According to a partial aspect, the system may have the (i) measuring unit that measures the state of cavitation in the dermis of the forehead of the subject, the (ii) analysis unit that analyzes the measured state of cavitation, the (iii) wrinkle evaluating unit that evaluates wrinkles based on the analysis results and the (iv) results output unit that outputs the evaluation results, located in physically separated locations.

According to a partial aspect, the system may further include a future prediction unit that predicts the future state of wrinkles based on analysis results, either instead of or in addition to the wrinkle evaluating unit of (iii) above.

According to a partial aspect, the system comprises (i) measuring means configured so as to measure the state of cavitation in the dermis of the forehead of the subject, (ii) analysis means configured so as to analyze the measured state of cavitation, (iii) wrinkle evaluating means configured so as to evaluate wrinkles based on the analysis results and (iv) results output means configured so as to output the evaluation results.

According to a partial aspect, the system may further include future prediction means that predicts the future state of wrinkles based on analysis results, either instead of or in addition to the wrinkle evaluating means of (iii) above.

According to a partial aspect, the measuring unit may be configured so as to measure the state of cavitation based on an echo or laser microscope, but the method of measurement is not particularly restricted. According to a partial aspect, the wrinkle evaluating unit can carry out the evaluation by comparing the measured value or analysis results with a predetermined reference value, or by insertion into the predetermined calculation formula. The predetermined reference value may be set based on data such as state of cavitation, state of transient wrinkles, state of fixed wrinkles, age or gender collected from multiple subjects. For example, wrinkles may be evaluated by deciding on the presence or absence of cavitation simply using a fixed size as the reference. The formula for calculating the degree of transient wrinkles X from the degree of cavitation Y (mm) can be expressed as: $Y = -0.1X + 1.5$. The formula for calculating the degree of fixed wrinkles X from the degree of cavitation Y (mm) can be expressed as: $Y = -0.1X + 1.3$.

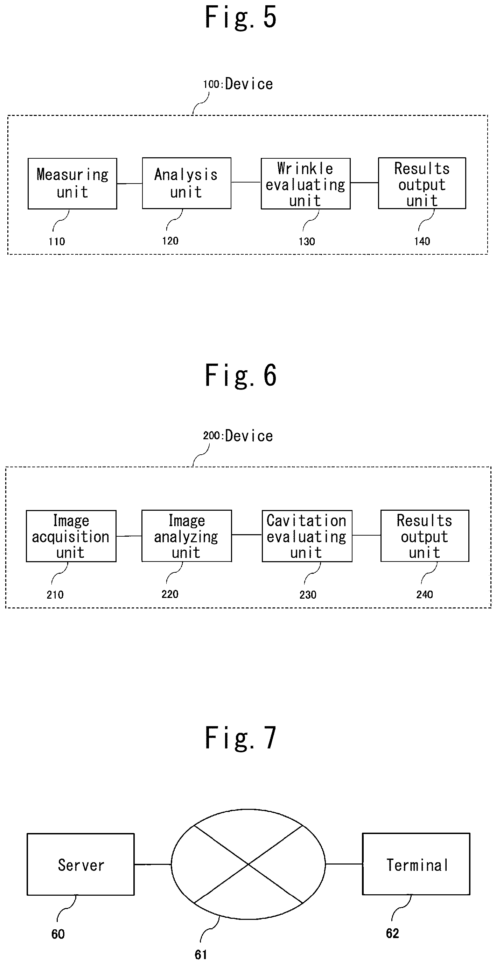

The device 100 depicted in Fig. 5 constitutes all or part of the system of the disclosure, and it has a measuring unit 110, an analysis unit 120, a wrinkle evaluating unit 130 and a results output unit 140. The device 100 has a computing device such as a CPU (Central Processing Unit), and a storage device such as ROM/RAM, hard disk or flash memory. The storage device is able to store a program for control of the device and data, and acquired data (images, parameters).

The computing device can cause the device 100 to function as the measuring unit 110, analysis unit 120, wrinkle evaluating unit 130 and results output unit 140, by executing the program stored in the storage device. That is, the measuring unit 110, analysis unit 120, wrinkle evaluating unit 130 and results output unit 140 may be constructed as functions of the CPU. The device 100 may also operate in cooperation with a computing device and storage device of a server connected by a network (such as a cloud server). The system of the disclosure may considered to be a system constructed with a server 60 and a terminal 62 connected to the server by a network 61 (Fig. 7).

The measuring unit 110 can receive information on cavitation, from an external source. The measuring unit 110 may also include an echo device or laser microscope for evaluation of cavitation. Alternatively, the measuring unit 110 may receive image data from an external device such as an echo device or laser microscope connected in a wired or wireless manner.

The analysis unit 120 can analyze inputted information, recognize cavitation in the dermis, and calculate the presence or absence and the size of cavitation. Any analysis method or machine learning method known to those skilled in the art may be used for analysis of the information, using a computer.

The wrinkle evaluating unit 130 evaluates wrinkles based on the data obtained from the analysis unit. For example, the wrinkle evaluating unit 130 may evaluate that the risk of wrinkle formation is high or that wrinkles are numerous, based on the presence or absence of dermal cavitation, or its size and/or number. Wrinkles may be transient wrinkles or fixed wrinkles. When numerous transient wrinkles are already visible, it may be evaluated that the risk of formation of fixed wrinkles is high.

According to an embodiment, the system may further include a future prediction unit that predicts the future state of wrinkles based on analysis results, either instead of or in addition to the wrinkle evaluating unit 130.

According to one embodiment, the wrinkle evaluating unit 130 may further include a recommendation unit which recommends a cosmetic, drug or cosmetic method depending on evaluation of wrinkles or the state of cavitation. The recommendation unit may also include a database (DB) that stores information relating to cosmetics, drugs or cosmetic methods. For the purpose of the present specification, the system of the disclosure which includes a recommendation unit may also be referred to as a "recommendation device".

The results output unit 140 may further include a display, printer and speaker for display of information to the user. The results output unit 140 may also output the results to an external device in a wired or wireless manner.

[Device and system for evaluation of cavitation]

One aspect of the disclosure relates to a system for evaluating cavitation, wherein cavitation is evaluated using the state of wrinkles on the forehead of a subject as the basis for assessment. According to a partial aspect, the system comprises (i) an image acquisition unit that acquires an image of wrinkles on the forehead of the subject, (ii) an image analyzing unit that analyzes the acquired image, (iii) a cavitation evaluating unit that evaluates the state of cavitation based on the analysis results and (iv) a results output unit that outputs the evaluation results. According to a partial aspect, the system may have the (i) image acquisition unit that acquires an image of wrinkles on the forehead of the subject, the (ii) image analyzing unit that analyzes the acquired image, the (iii) cavitation evaluating unit that evaluates the state of cavitation based on the analysis results and the (iv) results output unit that outputs the evaluation results, located in physically separated locations.

According to a partial aspect, the system comprises (i) image acquisition means configured so as to acquire an image of wrinkles on the forehead of the subject, (ii) image analysis means configured so as to analyze the acquired image, (iii) cavitation evaluating means configured so as to evaluate the state of cavitation based on the analysis results and (iv) results output means configured so as to output the evaluation results.

According to a partial aspect, the image acquisition unit can receive a skin image and/or shape from an external source. The image acquisition unit may also include hardware such as a camera which photographs images, illumination means, and a control processor. Alternatively, the image acquisition unit may receive image data from an external device such as a wired or wireless connected camera. For example, the system described in Japanese Patent No. 5528692 may be connected to the system of the disclosure in a wireless or wired manner, or it may be integrated as part of the system of the disclosure. The image acquisition unit may also include a database (DB) that stores inputted images and/or shapes.

According to a partial aspect, the image analyzing unit analyzes the inputted image and/or shape to allow evaluation of wrinkles on the forehead of the subject. Any image analysis method or machine learning method known to those skilled in the art may be used for analysis of the images and/or shapes, using a computer.

According to a partial aspect, the cavitation evaluating unit may carry out evaluation of cavitation by comparing the measured value for wrinkles with a predetermined reference value. The predetermined reference value may be set based on data such as state of cavitation, state of transient wrinkles, state of fixed wrinkles, age or gender collected from multiple subjects, for example. Cavitation may be evaluated, for example, by deciding on the presence or absence of wrinkles simply using a fixed size as the reference. Wrinkles may be transient wrinkles or fixed wrinkles.

The device 200 depicted in Fig. 6 constitutes all or part of the system of the disclosure, and it has an image acquisition unit 210, an image analyzing unit 220, a cavitation evaluating unit 230 and a results output unit 240. The device 200 has a computing device such as a CPU (Central Processing Unit), and a storage device such as ROM/RAM, hard disk or flash memory. The storage device is able to store a program for control of the device and data, and acquired data (images, parameters). The computing device can cause the device 200 to function as the image acquisition unit 210, image analyzing unit 220, cavitation evaluating unit 230 and results output unit 240, by executing the program stored in the storage device. That is, the image acquisition unit 210, image analyzing unit 220, cavitation evaluating unit 230 and results output unit 240 may be constructed as functions of the CPU. The device 200 may also operate in cooperation with a computing device and storage device of a server connected by a network (such as a cloud server). The system of the disclosure may be considered to be a system constructed with a server 60 and a terminal 62 connected to the server by a network 61 (Fig. 7).

The image acquisition unit 210 can receive images of wrinkles from an external source. The image acquisition unit 210 may also include hardware such as a camera which photographs images, illumination means, and a control processor, as explained above. Alternatively, the image acquisition unit 210 may receive data from an external device connected in a wired or wireless manner.

The image analyzing unit 220 analyzes the inputted information to allow evaluation of wrinkles on the forehead of the subject. Any analysis method or machine learning method known to those skilled in the art may be used for analysis of the information, using a computer.

The cavitation evaluating unit 230 evaluates cavitation based on the data obtained from the analysis unit. For example, the cavitation evaluating unit 230 may carry out evaluation of cavitation by comparing the measured value for wrinkles with a predetermined reference value, as explained above. The predetermined reference value may be set based on data such as state of cavitation, state of transient wrinkles, state of fixed wrinkles, age or gender collected from multiple subjects, for example. Cavitation may be evaluated, for example, by deciding on the presence or absence of wrinkles simply using a fixed size as the reference. Wrinkles may be transient wrinkles or fixed wrinkles.

According to one embodiment, the cavitation evaluating unit 230 may further include a recommendation unit which recommends a cosmetic, drug or cosmetic method depending on evaluation of wrinkles or the state of cavitation. The recommendation unit may also include a database (DB) that stores information relating to cosmetics, drugs or cosmetic methods. For the purpose of the present specification, the system of the disclosure which includes a recommendation unit may also be referred to as a "recommendation device".

The results output unit 240 may further include a display, printer and speaker for display of information to the user. The results output unit 240 may also output the results to an external device in a wired or wireless manner.

[Hardware configuration]

In the device described above, processing may be carried out by creating an execution program that can execute each function with a computer, and installing the execution program in a general personal computer or server, for example, or a combination thereof.

An example of the hardware configuration of a computer that can carry out processing according to the disclosure will now be explained with reference to the accompanying drawings.

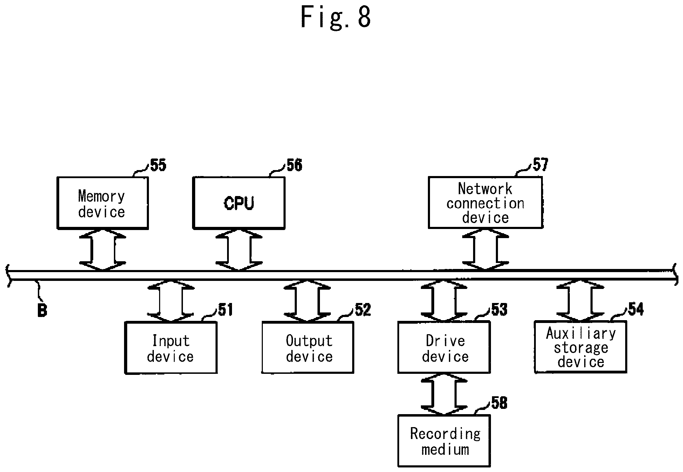

Fig. 8 is a diagram showing an example of a hardware configuration that can carry out processing according to the present disclosure. The computer body shown in Fig. 8 is constructed so as to comprise an input device 51, an output device 52, a drive device 53, an auxiliary storage device 54, a memory device 55, a CPU (Central Processing Unit) 56 which carries out various control operations, and a network connection device 57, all of which are mutually connected with a system bus B.

The input device 51 has a keyboard and a pointing device such as a mouse which are operated by a user, with different operation signals for program execution, for example, being inputted by the user. The input device 51 may also input various data such as facial images of a subject obtained from an external device connected to the network connection device 57, via a communication network.

The output device 52 has a display which displays windows or data necessary for operation of the computer body for processing according to the disclosure, allowing display of the process or results of program execution by the control program in the CPU 56. The output device 52 can also print the processing results on a print medium such as paper for presentation to the user.

The execution program installed in the computer body according to the disclosure is provided, for example, by a recording medium 58, such as a USB (Universal Serial Bus) memory, or a CD-ROM or DVD. The recording medium 58 in which the program is recorded may be set in the drive device 53, the execution program in the recording medium 58 being installed in the auxiliary storage device 54 from the recording medium 58 through the drive device 53.

The auxiliary storage device 54 is storage means such as a hard disk, and it can store the execution program of the disclosure, or a control program provided in a computer, with input/output as necessary.

The memory device 55 stores an execution program read out from the auxiliary storage device 54 by the CPU 56. The memory device 55 consists of a ROM (Read Only Memory) or RAM (Random Access Memory), for example.

All or part of the execution program may also be downloaded from an external device as necessary, via an optional network. An auxiliary storage device 54 in which the execution program is installed and/or a memory device 55 storing the installed execution program, may also be provided in an external device. The external device may be a server. The server may be a specialized server or an undefined server such as a cloud infrastructure. The execution program may be provided by a subscription-type service. A subscription-type service is one form of computer software usage, as a service which is paid for during the period in which the software is used. The manner of usage of the execution program may be in any form, with no particular restrictions.

The CPU 56 can control processing of the entire computer to carry out processing, including computing and input/output of data to the hardware configuration units, based on a control program on an OS (Operating System) and the execution program stored in the memory device 55. The necessary information during execution of the program can be acquired from the auxiliary storage device 54, and the results of execution may be stored.

The network connection device 57 can connect with a communication network or the like to obtain the execution program from another terminal or a server connected to the communication network, or to provide the obtained program execution results or the execution program itself to another terminal or server.

The analysis processing of the disclosure can be executed by such a hardware configuration. Installing the program allows processing according to the disclosure to be easily executed on a general personal computer, smartphone or tablet.

[Program]

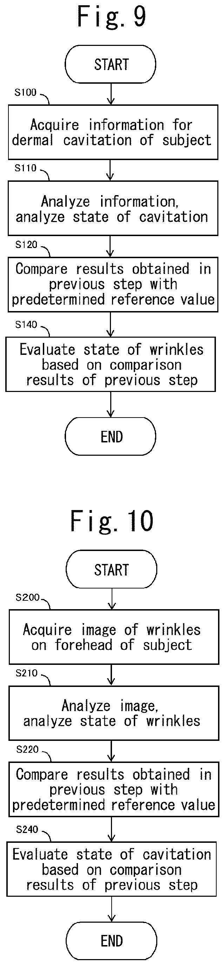

One partial aspect of the disclosure relates to a program that causes the method of the disclosure to be executed on a computer. Stated differently, the program of the disclosure may be a method that is fully or partially executed on a computer. According to a partial aspect, the program of the disclosure may be a program that executes on a computer a process which includes: (i) a step of acquiring information on cavitation in the dermis of the forehead of a subject (step 100), (ii) a step of analyzing the information and analyzing the state of cavitation (step 110), (iii) a step of comparing the analysis result obtained in step (ii) with a predetermined reference value or inserting the result into a predetermined formula (step 120), and (iv) a step of evaluating the condition of wrinkles based on the comparison result or calculated result of step (iii) (step 130). Fig. 9 shows an example of processing with a device. Steps 100 to 130 are carried out by executing a program by a CPU of the device (optionally including a server).

According to a partial aspect, the program may execute a step of predicting the future state of wrinkles based on analysis results, either instead of or in addition to steps (iii) and (iv).

A partial aspect of the disclosure relates to a computer-readable medium storing in a non-transitory manner a command to execute the following steps by a processor: (i) a step of acquiring information on cavitation in the dermis of the forehead of the subject (step 100), (ii) a step of analyzing the information and analyzing the state of cavitation (step 110), (iii) a step of comparing the analysis result obtained in step (ii) with a predetermined reference value or inserting the result into a predetermined formula (step 120), and (iv) a step of evaluating the condition of wrinkles based on the comparison result or calculated result of step (iii) (step 130).

According to a partial aspect, the computer-readable medium may store a command to execute a step of predicting the future state of wrinkles based on analysis results, either instead of or in addition to steps (iii) and (iv).

According to a partial aspect, in step 100, the program of the disclosure acquires information regarding cavitation in the dermis of the forehead of the subject. The information may be, but is not limited to, measured values from an echo or laser microscope, for example.

According to a partial aspect, the program of the disclosure analyzes inputted information in step 110, and calculates the presence or absence of cavitation and its size and/or number. Any image analysis method or machine learning method known to those skilled in the art may be used for analysis of the information, using a computer.

According to a partial aspect, in step 120 the program of the disclosure compares the presence or absence of cavitation and its size and/or number with a predetermined reference value. The predetermined reference value may be set based on data such as state of cavitation, state of transient wrinkles, state of fixed wrinkles, age or gender collected from multiple subjects, for example. Alternatively in step 120, the degree of cavitation may be entered into a predetermined formula to calculate the state of wrinkles.

According to a partial aspect, the program of the disclosure evaluates the state of wrinkles in step 130 based on the comparison result in step 120. For example, wrinkles may be evaluated by deciding on the presence or absence of cavitation simply using a fixed size as the reference. Alternatively, for example, it may evaluate that the risk of wrinkle formation is high or that wrinkles are numerous, based on the size of dermal cavitation, and/or its number. Wrinkles may be transient wrinkles or fixed wrinkles. When numerous transient wrinkles are already visible, it may be evaluated that the risk of formation of fixed wrinkles is high. Alternatively in step 130, the state of wrinkles may be evaluated on a 5-level scale of 0 to 4, for example, based on the calculation result of step 120.

According to a partial aspect, the program of the disclosure may further include in step 130 a step of recommending an appropriate cosmetic, drug or cosmetic method, based on evaluation of the state of cavitation. In this case, the program of the disclosure refers to a database (DB) storing information for cosmetics, drugs and cosmetic methods.

According to a partial aspect, the program of the disclosure may further include, in step 140, outputting the obtained evaluation results or recommendation results. Output of the results may be via any of different devices (such as a display, printer or speaker) connected in a wired or wireless manner.

According to a partial aspect, the program of the disclosure may be a program that executes on a computer a process which includes: (i) a step of acquiring an image of wrinkles in the dermis of the forehead of a subject (step 200), (ii) a step of analyzing the image and analyzing the state of wrinkles (step 210), (iii) a step of comparing the analysis results obtained in step (ii) with a predetermined reference value or inserting the results into a predetermined formula (step 220), and (iv) a step of evaluating the state of cavitation based on the comparison results or calculated results of step (iii) (step 230). Fig. 10 shows an example of processing with a device. Steps 200 to 230 are carried out by executing a program by a CPU of the device (optionally including a server).

A partial aspect of the disclosure relates to a computer-readable medium storing in a non-transitory manner a command to carry out the following steps executed by a processor: (i) a step of acquiring an image of wrinkles in the dermis of the forehead of the subject (step 200), (ii) a step of analyzing the image and analyzing the state of wrinkles (step 210), (iii) a step of comparing the analysis results obtained in step (ii) with a predetermined reference value or inserting the results into a predetermined formula (step 220), and (iv) a step of evaluating the state of cavitation based on the comparison results or calculated results of step (iii) (step 230).

According to a partial aspect, in step 200, the program of the disclosure acquires an image and/or shape of wrinkles in the dermis of the forehead of the subject. The image data may be acquired from an external device such as a wired or wireless connected camera, for example.

According to a partial aspect, the program of the disclosure analyzes an inputted image in step 210, and calculates the presence or absence of wrinkles and their sizes and/or number. Any known image analysis method or machine learning method may be used for analysis of the images.

According to a partial aspect, in step 220 the program of the disclosure compares the presence or absence of wrinkles and their size and/or number with a predetermined reference value. The predetermined reference value may be set based on data such as state of cavitation, state of transient wrinkles, state of fixed wrinkles, age or gender collected from multiple subjects, for example.

According to a partial aspect, the program of the disclosure evaluates the state of cavitation in step 230 based on the comparison results in step 220. For example, cavitation may be evaluated by deciding on the presence or absence of wrinkles simply using a fixed size as the reference. Alternatively, for example, it may evaluate the state of cavitation based on the sizes of wrinkles, and/or their number. Wrinkles may be transient wrinkles or fixed wrinkles.

According to a partial aspect, the program of the disclosure may further include in step 230 a step of recommending an appropriate cosmetic, drug or cosmetic method, based on evaluation of the estimated state of cavitation. In this case, the program of the disclosure refers to a database (DB) storing information for cosmetics, drugs and cosmetic methods.

According to a partial aspect, the program of the disclosure may further include, in step 240, outputting the obtained evaluation results or recommendation results. Output of the results may be via any of different devices (such as a display, printer or speaker) connected in a wired or wireless manner.

According to a partial aspect, the program of the disclosure may be installed in a smartphone or tablet for use. In this case, partial processing may also be carried out on a server connected via a network. According to another partial aspect, the program of the disclosure may be installed on the server for use. In this case, the image data may be acquired from a smartphone or tablet connected through the network, and the evaluation results may be outputted to the smartphone or tablet connected through the network.

EXAMPLES

Observation of dermal cavitation

The internal structure of the dermis layer of the forehead of a subject was observed in a noninvasive manner by ultrasound. A Prosound Alpha 5 (Aloka Co., Ltd., Tokyo, Japan), as a 13 MHz probe, was applied perpendicular to the skin that was covered with a thin-layer of ultrasonic gel, and the skin structure was measured using B mode. The shortest distance from the epidermis to the adipose layer was measured using Image J, and the sign-inverted value (mm) was recorded as the extent of dermal cavitation.

Fig. 1 shows the results of observation of a middle aged woman. As shown at right in Fig. 1, the results indicated cavitation with aging of the forehead skin.

Increase in transient wrinkles by cavitation

Fig. 2 shows the results of examining correlation between the degree of transient wrinkles and cavitation (fat infiltration) in the forehead of a subject. Transient wrinkles were defined as wrinkles observed on the forehead when the eyes were directed to look upwards (as evaluated by trained judges on a 5-level scale from 0 to 4). The data in Fig. 2 indicate that a significant correlation exists between the degree of transient wrinkles and the degree of cavitation (correlation coefficient R = -0.6; P < 0.001), and that the relationship between the degree of cavitation Y (mm) and the degree of transient wrinkles X can be expressed as Y = -0.1X + 1.5. These results demonstrate that by examining the degree of dermal cavitation of a subject it is possible to estimate the degree of transient wrinkles, and that conversely by examining the degree of transient wrinkles of a subject it is possible to estimate the degree of dermal cavitation.

Correlation between transient wrinkles and fixed wrinkles

Fig. 3 shows the results of examining correlation between transient wrinkles and fixed wrinkles in the forehead of a subject. Fixed wrinkles were defined as wrinkles observed on the forehead when the eyes were closed (as evaluated by trained judges on a 6-level scale from 0 to 5). The data in Fig. 3 indicate that increased transient wrinkles results in increased fixed wrinkles as well (correlation coefficient R = 0.9; P < 0.001). The relationship between the degree of fixed wrinkles Y and the degree of transient wrinkles X can be expressed as Y = -0.1X - 0.2.

Increase in fixed wrinkles by cavitation

Fig. 4 shows the results of examining correlation between the degree of fixed wrinkles and cavitation (fat infiltration) in the forehead of a subject. The data in Fig. 4 indicate that a significant correlation exists between the degree of fixed wrinkles and the degree of cavitation (correlation coefficient R = -0.6; P < 0.01), and that the relationship between the degree of cavitation Y (mm) and the degree of fixed wrinkles X can be expressed as: Y = -0.1X + 1.3. These results demonstrate that by examining the degree of dermal cavitation of a subject it is possible to estimate the degree of fixed wrinkles, and that conversely by examining the degree of fixed wrinkles of a subject it is possible to estimate the degree of dermal cavitation.

Relationship between age and degree of cavitation

As a result of analyzing the foreheads of healthy female subjects (aged 30 to 50, N=30), a significant correlation was also found between age and cavitation (correlation coefficient R = - 0.7; P < 0.01). The relationship between cavitation Y and age X can be expressed as: Y = -0.02X + 2.2 (P < 0.01). These results demonstrated that the degree of cavitation Y (mm) can be calculated from information for age X.

All of the publications mentioned throughout the present specification are incorporated herein in their entirety by reference. The technical scope of the disclosure is limited solely by the content of the present Claims, whereas the Examples described herein do not restrict the technical scope in any way. Modifications of the invention, such as additions, deletions or substitutions to the constituent features of the disclosure, are possible so long as the gist of the disclosure is maintained.

REFERENCE SIGNS LIST

51 Input device
52 Output device
53 Drive device
54 Auxiliary storage device
55 Memory device
56 CPU
57 Network connection device
58 Recording medium
60 Server
61 Network
62 Terminal
100 Device
110 Measuring unit

120 Analysis unit
130 Wrinkle evaluating unit
140 Results output unit
200 Device
210 Image acquisition unit
220 Image analyzing unit
230 Cavitation evaluating unit
240 Results output unit

**Claims**

1. A method for evaluating wrinkles, wherein the state of cavitation (fat infiltration) in the dermis of the forehead of a subject is used as the basis for assessment.

2. The method according to claim 1, comprising a step of measuring the state of cavitation by echo or laser microscope.

3. The method according to claim 1 or 2, wherein it is evaluated that the risk of wrinkle formation is high or that wrinkles are numerous when cavitation has occurred.

4. The method according to any one of claims 1 to 3, wherein the wrinkles are transient wrinkles and/or fixed wrinkles.

5. The method according to any one of claims 1 to 4, wherein cavitation is evaluated by the distance from the skin surface to a cavity, as measured by echo or laser microscope.

6. A system for evaluating wrinkles, wherein the wrinkles are evaluated using the state of cavitation (fat infiltration) in the dermis of the forehead of a subject as the basis for assessment.

7. The system according to claim 6, comprising:

   a measuring unit that measures the state of cavitation in the dermis of the forehead of the subject,
   an analysis unit that analyzes the measured state of cavitation,
   a wrinkle evaluating unit that evaluates wrinkles based on the analysis results and
   a results output unit that outputs the evaluation results.

8. The system according to claim 7, wherein the measuring unit is configured so as to measure the state of cavitation by echo or laser microscope.

9. The system according to claim 7 or 8, wherein the wrinkle evaluating unit carries out evaluation by comparing the measured value with a predetermined reference value.

10. A computer program for executing the method according to any one of claims 1 to 5 in a system according to any one of claims 7 to 9.

11. A method for evaluating cavitation (fat infiltration), wherein the state of wrinkles on the forehead of a subject is used as the basis for assessment.

12. The method according to claim 11, wherein the wrinkles are transient wrinkles and/or fixed wrinkles.

13. The method according to claim 11 or 12, wherein it is evaluated that cavitation has occurred when wrinkles have formed.

14. A system for evaluating cavitation (fat infiltration), wherein the state of cavitation is evaluated using the state of wrinkles on the forehead of a subject as the basis for assessment.

15. The system according to claim 14, comprising:

   an image acquisition unit that acquires an image of wrinkles on the forehead of a subject,

an image analyzing unit that analyzes the acquired image,
a cavitation evaluating unit that evaluates the state of cavitation based on the analysis results and
a results output unit that outputs the evaluation results.

**16.** The system according to claim 15, wherein the cavitation evaluating unit carries out evaluation by comparing the measured value with a predetermined reference value.

**17.** A computer program for executing the method according to any one of claims 11 to 13 in a system according to any one of claims 14 to 16.

**18.** A skin sensing method for evaluation of wrinkles, wherein the state of cavitation (fat infiltration) in the dermis of the forehead of a subject is included as a measured parameter.

**19.** A recommendation method based on evaluation of wrinkles using sensing data obtained by the method according to claim 18.

**20.** A method for predicting future cavitation state and/or wrinkle state, based on the state of cavitation (fat infiltration) in the dermis of the forehead of a subject at a measuring time point.

# Fig. 1

No cavitation          cavitation

Cavitation index:
Minimum depth of fat

Dermis layer

Subcutaneous fat

# Fig. 2

Y = -0.1 X + 1.5
R = -0.6 (P<0.001)

Degree of cavitation (mm)

Degree of transient wrinkles

# Fig. 3

Y = -1.0 X – 0.2
R = 0.9 (P<0.001)

Degree of fixed wrinkles (y-axis)

Degree of transient wrinkles (x-axis)

# Fig. 4

Y = -0.1 X + 1.3
R = -0.6 (P<0.01)

Degree of cavitation (mm) (y-axis)

Degree of fixed wrinkles (x-axis)

# Fig. 5

100:Device

| Measuring unit | Analysis unit | Wrinkle evaluating unit | Results output unit |
|---|---|---|---|

110     120     130     140

# Fig. 6

200:Device

| Image acquisition unit | Image analyzing unit | Cavitation evaluating unit | Results output unit |
|---|---|---|---|

210     220     230     240

# Fig. 7

| Server | | Terminal |
|---|---|---|

60     61     62

# Fig. 8

# Fig. 9

```
        ( START )
           |
S100       ↓
  ┌─────────────────────────┐
  │   Acquire information for │
  │ dermal cavitation of subject │
  └─────────────────────────┘
           |
S110       ↓
  ┌─────────────────────────┐
  │    Analyze information,   │
  │  analyze state of cavitation │
  └─────────────────────────┘
           |
S120       ↓
  ┌─────────────────────────┐
  │ Compare results obtained in │
  │      previous step with    │
  │ predetermined reference value │
  └─────────────────────────┘
           |
S140       ↓
  ┌─────────────────────────┐
  │  Evaluate state of wrinkles │
  │     based on comparison   │
  │    results of previous step │
  └─────────────────────────┘
           |
           ↓
        ( END )
```

# Fig. 10

```
        ( START )
           |
S200       ↓
  ┌─────────────────────────┐
  │   Acquire image of wrinkles │
  │     on forehead of subject  │
  └─────────────────────────┘
           |
S210       ↓
  ┌─────────────────────────┐
  │      Analyze image,       │
  │   analyze state of wrinkles │
  └─────────────────────────┘
           |
S220       ↓
  ┌─────────────────────────┐
  │ Compare results obtained in │
  │      previous step with    │
  │ predetermined reference value │
  └─────────────────────────┘
           |
S240       ↓
  ┌─────────────────────────┐
  │ Evaluate state of cavitation │
  │     based on comparison   │
  │    results of previous step │
  └─────────────────────────┘
           |
           ↓
        ( END )
```

## INTERNATIONAL SEARCH REPORT

| International application No. |
|---|
| **PCT/JP2022/044536** |

**A. CLASSIFICATION OF SUBJECT MATTER**

*A61B 5/107*(2006.01)i; *A61B 5/00*(2006.01)i; *A61B 8/14*(2006.01)i
FI: A61B5/107 800; A61B5/00 M; A61B8/14

According to International Patent Classification (IPC) or to both national classification and IPC

**B. FIELDS SEARCHED**

Minimum documentation searched (classification system followed by classification symbols)

A61B5/107; A61B5/00; A61B8/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2010-148548 A (KAO CORP) 08 July 2010 (2010-07-08)<br>paragraphs [0021]-[0031], fig. 1-9 | 1-20 |
| A | JP 2010-119431 A (SHISEIDO CO LTD) 03 June 2010 (2010-06-03)<br>paragraphs [0032]-[0124], fig. 1-16 | 1-20 |
| A | JP 2002-330943 A (POLA CHEM IND INC) 19 November 2002 (2002-11-19)<br>paragraphs [0007]-[0013], fig. 1-4 | 1-20 |
| A | JP 2020-171489 A (POLA CHEM IND INC) 22 October 2020 (2020-10-22)<br>paragraphs [0016]-[0061], fig. 1-8 | 1-20 |
| A | JP 2012-161371 A (SHISEIDO CO LTD) 30 August 2012 (2012-08-30)<br>paragraphs [0012]-[0031], fig. 1-3 | 1-20 |
| A | WO 2017/022091 A1 (SHISEIDO CO LTD) 09 February 2017 (2017-02-09)<br>paragraphs [0022]-[0023], [0033]-[0035], fig. 1-2 | 1-20 |
| A | JP 2016-174791 A (HITACHI MAXELL) 06 October 2016 (2016-10-06)<br>paragraphs [0029]-[0109], fig. 1-9 | 19 |

☐ Further documents are listed in the continuation of Box C.     ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **03 February 2023** | **14 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/044536**

| Patent document cited in search report | | | Publication date (day/month/year) | Patent family member(s) | Publication date (day/month/year) |
|---|---|---|---|---|---|
| JP | 2010-148548 | A | 08 July 2010 | (Family: none) | |
| JP | 2010-119431 | A | 03 June 2010 | (Family: none) | |
| JP | 2002-330943 | A | 19 November 2002 | (Family: none) | |
| JP | 2020-171489 | A | 22 October 2020 | (Family: none) | |
| JP | 2012-161371 | A | 30 August 2012 | (Family: none) | |
| WO | 2017/022091 | A1 | 09 February 2017 | US 2018/0200179 A1<br>paragraphs [0074]-[0075],<br>[0085]-[0086], fig. 1-2<br>EP 3332765 A1<br>CN 107847424 A<br>KR 10-2018-0030850 A | |
| JP | 2016-174791 | A | 06 October 2016 | (Family: none) | |

Form PCT/ISA/210 (patent family annex) (January 2015)

**EP 4 449 988 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016216435 A
- JP 2010115131 A
- JP 2013116088 A
- WO 2017022091 A
- JP 5528692 B